(19) Europäisches Patentamt
European Patent Office
Office européen des brevets

(11) Veröffentlichungsnummer: **0 563 831 B1**

(12) # EUROPÄISCHE PATENTSCHRIFT

(45) Veröffentlichungstag der Patentschrift: **13.09.95**

(51) Int. Cl.$^{6}$: **C07C 53/02**, C07C 51/02

(21) Anmeldenummer: **93105073.6**

(22) Anmeldetag: **27.03.93**

(54) **Verfahren zur Herstellung von Ameisensäure durch thermische Spaltung von quartären Ammoniumformiaten.**

(30) Priorität: **03.04.92 DE 4211141**

(43) Veröffentlichungstag der Anmeldung:
**06.10.93 Patentblatt 93/40**

(45) Bekanntmachung des Hinweises auf die Patenterteilung:
**13.09.95 Patentblatt 95/37**

(84) Benannte Vertragsstaaten:
**BE CH DE FR GB IT LI NL**

(56) Entgegenhaltungen:
**EP-A- 0 156 309**
**EP-A- 0 161 544**
**DE-C- 2 545 658**

(73) Patentinhaber: **BASF Aktiengesellschaft**
**Carl-Bosch-Strasse 38**
**D-67063 Ludwigshafen (DE)**

(72) Erfinder: **Kiefer, Hans, Dr.**
**Im Biengarten 25**
**W-6730 Neustadt (DE)**
Erfinder: **Hupfer, Leopold, Dr.**
**Waltershoehe 3**
**W-6701 Friedelsheim (DE)**
Erfinder: **Lippert, Ferdinand, Dr.**
**Salinenstrasse 88**
**W-6702 Bad Duerkheim (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

## Beschreibung

Die vorliegende Erfindung betrifft ein verbessertes Verfahren zur Herstellung von Ameisensäure durch thermische Spaltung von quartären Ammoniumformiaten in Gegenwart von bestimmten Formamiden.

Aus der EP-A-1 432 ist bekannt, daß substituierte N-Alkylimidazole die Freisetzung, d.h. die Abtrennung der Ameisensäure aus Trialkylformiaten erleichtern. Die hochsiedenden N-Alkylimidazole sind jedoch bei thermischer Dauerbelastung in Anwesenheit von Ameisensäure nicht ausreichend stabil und bedürfen eines hohen Energieaufwandes bei der Abtrennung. Ferner verfärbt sich die so erhaltene Ameisensäure beim Stehen.

Der vorliegenden Erfindung lag daher die Aufgabe zugrunde, den zuvor genannten Nachteilen abzuhelfen.

Demgemäß wurde ein neues und verbessertes Verfahren zur Herstellung von Ameisensäure durch thermische Spaltung von quartären Ammoniumformiaten der allgemeinen Formel I

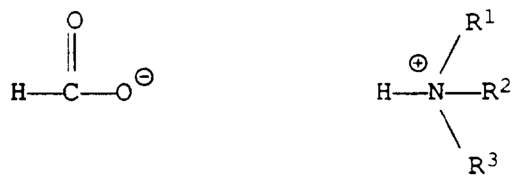

(I),

in der

$R^1, R^2, R^3$ $C_1$- bis $C_{14}$-Alkyl, $C_3$- bis $C_8$-Cycloalkyl, Aryl und $C_7$- bis $C_{16}$-Aralkyl oder gemeinsam eine gegebenenfalls durch ein- bis vierfach $C_1$- bis $C_4$-Alkyl substituierte 1,4- oder 1,5-Alkylengruppe bedeutet, mit der Maßgabe, daß die Summe der Kohlenstoffatome von $R^1$, $R^2$ und $R^3$ in den quartären Ammoniumformiaten I 7 bis 40 beträgt,

gefunden, welches dadurch gekennzeichnet ist, daß man die Spaltung in Anwesenheit von sekundären Formamiden der allgemeinen Formel II

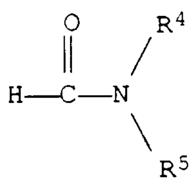

(II),

in der

$R^4$ und $R^5$ $C_2$- bis $C_{10}$-Alkyl, $C_3$- bis $C_8$-Cycloalkyl, Aryl und $C_7$- bis $C_{16}$-Aralkyl oder gemeinsam eine gegebenenfalls durch ein- bis vierfach $C_1$- bis $C_4$-Alkyl substituierte 1,4- oder 1,5-Alkylengruppe bedeutet,

durchführt, die 30 bis 150 °C niedriger sieden als das im Formiat I enthaltene tertiäre Amin der allgemeinen Formel III

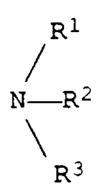

(III),

in der $R^1$, $R^2$ und $R^3$ die oben genannten Bedeutungen haben.

Das erfindungsgemäße Verfahren kann wie folgt durchgeführt werden:
Die zur thermischen Spaltung (Zersetzung) eingesetzten quartären Ammoniumformiate I können

a) durch direkte Umsetzungen der entsprechenden Amine mit Ameisensäure gemäß GB-A-1 028 930 bzw. US-A-3 414 610 oder

b) durch übergangsmetallkatalysierte Hydrierung von Kohlendioxid zu Ameisensäure in Gegenwart einer Stickstoff-Base wie in EP-A-95 321, EP-A-151 510 oder EP-A-357 243 beschrieben oder

c) durch Umsetzung von Methylformiat und Wasser und anschließende Extraktion der gebildeten Ameisensäure mit einem tertiären Amin (DE-A-34 28 319) oder mit Formamiden wie in US-A-4 076 594, US-A-4 262 140 bzw. US-A-4 326 073 oder

d) durch Umsetzung von Methylformiat und Wasser in Gegenwart von Imidazolderivaten (EP-A-1 432), Formamiden (EP-A-161 544) oder mit den tertiären Aminen III bei Temperaturen von 50 bis 200 °C, bevorzugt 100 bis 150 °C, besonders bevorzugt 110 bis 140 °C und Drücken von 1 bis 50 bar, bevorzugt 1 bis 20 bar, besonders bevorzugt 5 bis 15 bar erhalten werden.

Die eingesetzten Gemische (Rohgemische) können je nach Herstellverfahren neben dem quartären Ammoniumformiat I weitere Verbindungen wie z.B. Lösungsmittel, nicht umgesetzte Reaktionspartner, gebildete Koppelprodukte und/oder Katalysatorkomponenten enthalten. In aller Regel enthalten z.B. nach Verfahren d) diese Rohgemische neben dem quartären Ammoniumformiat I als weitere Verbindungen z.B. noch Wasser, Methanol und Methylformiat, die sich weitgehend bei Normaldruck abdestillieren lassen.

Die erfindungsgemäße thermische Spaltung der quartären Ammoniumformiate I läßt sich bei Drücken von 0,01 bis 2 bar, bevorzugt 0,02 bis 1 bar, besonders bevorzugt 0,05 bis 0,5 bar destillativ durchführen. Die Destillationstemperatur hängt dabei im wesentlichen von dem eingestellten Druck ab.

Zur thermischen Spaltung eignen sich vor allem Destillationsapparaturen wie Destillationskolonnen, z.B. Füllkörper- und Glockenbodenkolonnen. Als Füllkörper eignen sich z.B. Packungen, Glas-Raschig- und Pall-Ringe und zur Vermeidung von Korrosion vorzugsweise keramische Füllkörper.

Bei der technischen Ausgestaltung dieses neuen Verfahrens geht man im Falle der Methylformiat-Hydrolyse beispielsweise so vor, daß man die Hydrolyse nach EP-A-1 432 in Gegenwart der Base und Wasser durchführt. Nach beendeter Hydrolyse setzt man das substituierte Formamid zu und arbeitet das Gemisch destillativ auf (Abtrennen des restlichen Methylformiats, Wassers und des gebildeten Methanols). Anschließend trennt man in einer Destillationsblase im Vakuum (vorteilhaft bis ca. 150 Torr) die wasserfreie oder weitgehend wasserfreie Ameisensäure über Kopf in reiner Form ab. Das zugesetzte Formamid kann anschließend, wenn gewünscht, destillativ von der freien Base abgetrennt werden. Man kann jedoch auch das im Sumpf der Base zurückbleibende Gemisch aus Base und Formamid ohne Trennung in die Hydrolysestufe des Methylformiats zurückführen.

Andere Ausgestaltungen des Verfahrens sind ebenfalls möglich. So kann beispielsweise auch die Hydrolyse des Methylformiats zuerst ohne jeden Zusatz erfolgen. Zur Vervollständigung der Reaktion kann anschließend erst das Formamid und dann die Base oder beide gleichzeitig zugegeben werden.

Besonders vorteilhaft gestaltet sich das Verfahren bei kontinuierlicher Prozeßführung. Hier wird aus dem Austrag der Hydrolysereaktion - bestehend aus geringen Mengen Methylformiat und Wasser sowie Methanol, Ameisensäure und der Base - über Kopf Methylformiat, Methanol und Wasser bei Normaldruck abdestilliert. Der Sumpfabzug aus der ersten Kolonne wird einer zweiten, unter Vakuum stehenden Kolonne (50 bis 150 Torr) zugeführt. Zur leichteren Abtrennung der Ameisensäure enthält diese zweite Kolonne das erfindungsgemäße, substituierte Formamid.

Man wählt dieses Formamid vorzugsweise so aus, daß es sich nur auf den unteren Trennstufen dieser Kolonne befindet. Durch diese Maßnahme erreicht man, daß dieses Formamid aus dem Sumpf weitgehend ferngehalten wird und aus dem Sumpf die praktisch formamidfreie Base abgezogen und in die Hydrolysestufe zurückgeführt werden kann.

Über Kopf zieht man die wasserfreie bzw. weitgehend wasserfreie Ameisensäure in reiner Form ab.

Für den Erfolg des Verfahrens ist entscheidend, daß das zugesetzte Formamid eine höhere Flüchtigkeit als die N-Base besitzt. Aus der Vielzahl der möglichen Formamide wählt man bevorzugt eines aus mit einem um 30 bis 150 °C, bevorzugt 50 bis 120 °C, niedrigeren Siedepunkt im Vergleich zur N-Base.

Ein weiterer Vorteil des Zusatzes von substituierten Formamiden bei der thermischen Trennung besteht darin, daß tert.-Alkylamine mit niedrigeren Molekulargewichten für diesen Zweck eingesetzt werden können.

Die Substituenten $R^1$, $R^2$ und $R^3$ in den Verbindungen I und III sowie $R^4$ und $R^5$ in den Verbindungen II haben folgende Bedeutungen:

$R^1$,$R^2$,$R^3$,$R^4$,$R^5$

- $C_3$- bis $C_8$-Cycloalkyl wie Cyclopropyl, Cyclobutyl, Cyclopentyl, Cyclohexyl, Cycloheptyl und Cyclooctyl, bevorzugt Cyclopentyl, Cyclohexyl und Cyclooctyl,
- Aryl wie Phenyl, 1-Naphthyl, 2-Naphthyl, 1-Anthryl, 2-Anthryl und 9-Anthryl, bevorzugt Phenyl, 1-Naphthyl und 2-Naphthyl, besonders bevorzugt Phenyl,

- $C_7$- bis $C_{16}$-Aralkyl, bevorzugt $C_7$- bis $C_{12}$-Phenylalkyl wie Benzyl, 1-Phenethyl, 2-Phenethyl, 1-Phenyl-propyl, 2-Phenyl-propyl, 3-Phenyl-propyl, 1-Phenyl-butyl, 2-Phenyl-butyl, 3-Phenyl-butyl und 4-Phenyl-butyl, besonders bevorzugt Benzyl, 1-Phenethyl und 2-Phenethyl,

oder $R^1$ und $R^2$ oder $R^1$ und $R^3$ oder $R^2$ und $R^3$ gemeinsam

- eine gegebenenfalls durch ein- bis vierfach $C_1$- bis $C_4$-Alkyl substituierte 1,4- oder 1,5-Alkylengruppe mit der Maßgabe, daß die Summe der Kohlenstoffatome von $R^1$, $R^2$ und $R^3$ in den Verbindungen I und III 7 bis 40 beträgt, bevorzugt eine gegebenenfalls durch ein- bis vierfach $C_1$- bis $C_4$-Alkyl substituierte 1,4- oder 1,5-Alkylengruppe mit der Maßgabe, daß die Summe der Kohlenstoffatome von $R^1$, $R^2$ und $R^3$ in den Verbindungen I und III 10 bis 24 beträgt, wie $-(CH_2)_4-$, $-(CH_2)_5-$, $-CH(CH_3)-CH_2-CH_2-$, $-CH_2-CH(CH_3)-CH_2-$, ...

$R^1$,$R^2$,$R^3$

- $C_1$- bis $C_{14}$-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, n-Heptyl, iso-Heptyl, n-Octyl, iso-Octyl, n-Nonyl, iso-Nonyl, n-Decyl, iso-Decyl, n-Undecyl, iso-Undecyl, n-Dodecyl und iso-Dodecyl, bevorzugt $C_1$- bis $C_8$-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, n-Heptyl, iso-Heptyl, n-Octyl, iso-Octyl, besonders bevorzugt $C_1$- bis $C_4$-Alkyl wie Methyl, Ethyl, n-Propyl, iso-Propyl, n-Butyl, iso-Butyl, sec.-Butyl und tert.-Butyl, mit der Maßgabe, daß die Summe der Kohlenstoffatome von $R^1$, $R^2$ und $R^3$ 7 bis 40, bevorzugt 10 bis 24 beträgt,

$R^4$ und $R^5$

- $C_2$- bis $C_{10}$-Alkyl wie Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, n-Heptyl, iso-Heptyl, n-Octyl, iso-Octyl, n-Nonyl, iso-Nonyl, n-Decyl, iso-Decyl, n-Undecyl, iso-Undecyl, n-Dodecyl und iso-Dodecyl, bevorzugt $C_2$- bis $C_8$-Alkyl wie Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, n-Heptyl, iso-Heptyl, n-Octyl, iso-Octyl, besonders bevorzugt $C_2$- bis $C_4$-Alkyl wie Ethyl, n-Propyl, iso-Propyl, n-Butyl, iso-Butyl, sec.-Butyl und tert.-Butyl.

Als N-tert.-Alkylamine kommen insbesondere N-Tri-pentyl-, N-Tri-hexyl-, N-Tri-heptyl-, N-Tri-octylamin, Dimethyltetradecylamin, Diethyl-tetrahecyl-, Dimethyl-octyl-, Diethyl-octylamin oder auch N,N'-Dimorpholinoethan oder N-Hexyl-morpholin in Frage.

## Beispiele

### Beispiel 1 bis 4

Hydrolyse von Methylformiat mit tertiären Aminen

In 0,3-l-Rührautoklaven wurde der Methylformiat-Umsatz bei einem Molverhältnis Methylformiat : Wasser : Base = 1 : 1 : 1 und 120°C/bei einer Stunde Verweilzeit in Abhängigkeit von der Art der Base ermittelt.

| Base | Umsatz [%] |
|---|---|
| Dimethyltetradecylamin | 68 |
| Tri-n-octylamin | 68 |
| Dimethylcyclohexylamin | 78 |
| Tetramethylethylendiamin | 74 |

### Beispiel 5 bis 8

Destillative Gewinnung von Ameisensäure

In einer kontinuierlichen Destillationsapparatur, bestehend aus einer Glockenbodenkolonne (Kolonne I) (20 theor. Böden, Durchmesser: 5 cm) und einer Füllkörperkolonne (Kolonne II) (5 mm Glas-Raschig-Ringe, Höhe 130 cm, Durchmesser: 4 cm) wurden verschiedene Hydrolysate, bestehend aus Methylformiat, Methanol, Wasser, Ameisensäure und Base mit offener Fahrweise bezüglich Methylformiat, Methanol und

Ameisensäure (Wasser) und geschlossener Fahrweise bezüglich der Base in ihre Komponenten aufgetrennt.

Vergleichsbeispiel A:

Das den Hydrolysereaktor mit 130°C und 8 bar verlassende Gemisch aus 1025 g/h Tri-n-octylamin, 203 g/h Ameisensäure, 136 g/h Methanol, 100 g/h Methylformiat und 40 g/h Wasser wird der Kolonne I (Glockenbodenkolonne) auf dem 15. theoretischen Boden (von oben gezählt) zugeführt. Die Rektifikation erfolgt bei Normaldruck und einer Sumpftemperatur von 158°C. Die Kopftemperatur beträgt 55°C, das Rücklaufverhältnis beträgt 4:1. Das wasser- und ameisensäurefreie Kopfprodukt aus 129 g/h Methanol und 95 g/h Methylformiat wird abgezogen. Der Sumpf wird in die Mitte der Füllkörperkolonne II (Durchmesser: 4 cm, Höhe 130 cm, 5 mm Glas-Raschig-Ringe), die im Vakuum bei 100 mbar betrieben wird, zugeführt. Die thermische Spaltung des Ammoniumformiats wird bei einer Sumpftemperatur von 178°C durchgeführt. Bei einem Rücklaufverhältnis von R = 4 zieht man bei einer Kopftemperatur von 47°C stündlich ein Kopfprodukt von 150 g/g Ameisensäure und 31 g/h Wasser ab. Das Sumpfprodukt der Kolonne II, das aus 1024 g/h Tri-n-octylamin und 12 g/h Ameisensäure besteht, führt man in den Hydrolysereaktor zurück.

Die Ameisensäure enthält etwa 2361 ppm organische Verunreinigungen. Die Ameisensäureverluste liegen bei 20 %.

Vergleichsbeispiel B:

Auf analoge Weise wie in Beispiel 5 und mit den in Tabellen 1 und 2 angegebenen Einsatzmengenverhältnissen und Bedingungen wird das Verfahren mit Dimethyltetradecylamin anstelle von Tri-n-octylamin durchgeführt. Die Ameisensäureverluste betragen ca. 24 %, wobei die erhaltene Ameisensäure ca. 122 ppm organische Verunreinigungen enthält.

Beispiel 5

Bei der Durchführung des Verfahrens gemäß Vergleichsbeispiel A, jedoch mit Zusatz von Dibutylformamid werden mehrere Vorteile deutlich (siehe Tabellen 1 und 2). Bei vergleichbaren Bedingungen betrugen die Ameisensäureverluste nur ca. 0,5 %, die erhaltene Ameisensäurekonzentration 88 %. Es fanden sich lediglich 72 ppm organische Verunreinigungen in der Ameisensäure.

Beispiel 6

Wie in Beispiel 5 beschrieben, nur mit Dimethyltetradecylamin und Dibutylformamid als N-Basen. Bei ähnlichen Versuchsbedingungen (siehe Tabellen 1 und 2) lagen die Ameisensäureverluste bei 9 %, die erhaltene Ameisensäurekonzentration betrug 91,5 %. Es fanden sich lediglich 4 ppm organische Verunreinigungen gegenüber 122 ppm im Vergleichsbeispiel B.

Diese Beispiele verdeutlichen die für die Spaltung der Ammoniumformiate entscheidende, bisher unbekannte Wirkung der Formamide. Erst die Gegenwart dieser Zwischensieder ermöglicht die verlustfreie Gewinnung hochreiner Ameisensäure aus Ammoniumformiaten. Im Gegensatz zu dem in DE-A-34 28 319 beschriebenen Verfahren, in dem durch Zusatz von Kohlenwasserstoffen, die mit Wasser und Ameisensäure Heteroazeotrope bilden, die Abtrennung der Ameisensäure aus Ammoniumformiaten ermöglicht wird, muß in diesem neuen, erfindungsgemäßen Verfahren das Formamid nicht verdampft werden und kann als Zwischensieder in der Kolonne gehalten werden. Den Zulauf der Kolonne 2 wählt man deshalb je nach verwendetem Formamid oberhalb des Abtriebsteils der Kolonne.

Kontinuierliche Destillation der Hydrolysate

Tabelle 1: Destillationsbedingungen

| Beispiel | Base | RF | Kolonne 1 Sumpf [°C] | [Normaldruck] Kopf [°C] | Kolonne 2 Sumpf [°C] | [100 mbar] Kopf [°C] |
|---|---|---|---|---|---|---|
| A | TOA | [4:1] | 158 | 55 | 178 | 47 |
| 5 | TOA/DBF | [4:1] | 150 | 58 | 180 | 48 |
| B | DMC14N | [4:1] | 148 | 55 | 181 | 52 |
| 6 | DMC14N/DBF | [4:1] | 150 | 65 | 180 | 47 |

RF=Rückflußverhältnis; TOA=Tri-n-octylamin; DBF=Dibutylformamid, DMC14N=Dimethyltetradecylamin

Tabelle 2: Mengenströme

| Bsp. | Zulauf K 1 [g/h] | | | Dest K 1 [g/h] | | |
|---|---|---|---|---|---|---|
| | gesamt | Ameisensäure | Amin/DBF | gesamt | Methylformiat | MeOH |
| A | 1472 | 203 | 1025,2 | 222,4 | 95,3 | 118,9 |
| 5 | 891,81 | 116,4 | 575,9/64 | 137,2 | 57,8 | 76,8 |
| B | 1411,1 | 229,6 | 928 | 246,9 | 129,9 | 103,12 |
| 6 | 974,5 | 164 | 572,67/63,67 | 217,2 | 93,8 | 93,8 |

| Bsp. | Zulauf K 2 [g/h] | Dest K 2 [g/h] | | | Sumpf K 2 [g/h] | | |
|---|---|---|---|---|---|---|---|
| | | gesamt | Ameisensäure | $H_2O$ | gesamt | Ameisensäure | Amin |
| A | 1249,6 | 176 | 150 | 31 | 1036 | 12 | 1024 |
| 5 | 754,6 | 127 | 112 | 15 | 626 | 4,2 | 622 |
| B | 1164,2 | 169,1 | 152 | 16,6 | 959 | 19 | 928 |
| 6 | 757,3 | 150,6 | 135 | 15 | 575,2 | 9 | 566 |

Tabelle 3: Kontinuierliche Destillation der Hydrolysate (Zusammensetzung)

| Bsp. | Base | Zulauf | Zulauf Ameisensäure | Destillat-Kolonne 2 | | | Sumpf-Kolonne 2 | Verluste |
|---|---|---|---|---|---|---|---|---|
| | [1:0,25 mol/ mol] | [ml/h] | Konzentration [Gew.-%] | Ameisensäure [%] | $H_2O$ [%] | org. Verunr. [ppm] | Ameisensäure [%] | Ameisensäure [%] |
| A | TOA | 2000 | 83,5 | 83,2 | 16,8 | 2361 | 1,1 | 20,5 |
| 5 | TOA/DBF | 1250 | 88,9 | 88,1 | 11,9 | 72 | 0,7 | 0,5 |
| B | DMC14N | 2000 | 86,3 | 89,9 | 10 | 122 | 2,4 | 24,2 |
| 6 | DMC14N/DBF | 1250 | 85,9 | 91,5 | 9,5 | 4 | 1,5 | 9,4 |

TOA = Tri-n-octylamin
DMC14N = Dimethyltetradecylamin
DBF = Dibutylformamid
$H_2O$ = Wasser

## Patentansprüche

**1.** Verfahren zur Herstellung von Ameisensäure durch thermische Spaltung von quartären Ammoniumformiaten der allgemeinen Formel I

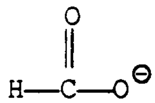 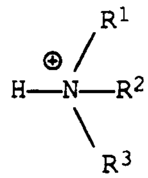 (I),

in der

$R^1$,$R^2$,$R^3$ $C_1$- bis $C_{14}$-Alkyl, $C_3$- bis $C_8$-Cycloalkyl, Aryl und $C_7$- bis $C_{16}$-Aralkyl oder gemeinsam eine gegebenenfalls durch ein- bis vierfach $C_1$- bis $C_4$-Alkyl substituierte 1,4- oder 1,5-Alkylengruppe bedeutet, mit der Maßgabe, daß die Summe der Kohlenstoffatome von $R^1$, $R^2$ und $R^3$ in den quartären Ammoniumformiaten I 7 bis 40 beträgt,

dadurch gekennzeichnet, daß man die Spaltung in Anwesenheit von sekundären Formamiden der allgemeinen Formel II

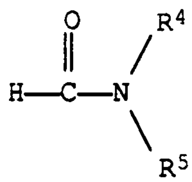 (II),

in der

$R^4$ und $R^5$ $C_2$- bis $C_{10}$-Alkyl, $C_3$- bis $C_8$-Cycloalkyl, Aryl und $C_7$- bis $C_{16}$-Aralkyl oder gemeinsam eine gegebenenfalls durch ein- bis vierfach $C_1$- bis $C_4$-Alkyl substituierte 1,4- oder 1,5-Alkylengruppe bedeutet, durchführt, die 30 bis 150 °C niedriger sieden als das im Formiat I enthaltene tertiäre Amin der allgemeinen Formel III

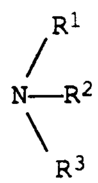 (III),

in der $R^1$, $R^2$ und $R^3$ die oben genannten Bedeutungen haben.

2. Verfahren zur Herstellung von Ameisensäure durch thermische Spaltung von quartären Ammoniumformiaten I nach Anspruch 1, dadurch gekennzeichnet, daß die Summe der Kohlenstoffatome von $R^1$, $R^2$ und $R^3$ in den quartären Ammoniumformiaten I 10 bis 24 beträgt.

3. Verfahren zur Herstellung von Ameisensäure durch thermische Spaltung von quartären Ammoniumformiaten I nach Anspruch 1, dadurch gekennzeichnet, daß man als sekundäres Formamid II N-Dibutylformamid verwendet.

4. Verfahren zur Herstellung von Ameisensäure durch thermische Spaltung von quartären Ammoniumformiaten I nach Anspruch 1, dadurch gekennzeichnet, daß man quartäre Ammoniumformiate I der tertiären Amine III Trihexylamin, Triheptylamin, Trioctylamin, Dimethyltetradecylamin,N-Hexylmorpholin oder N,N'-Dimorpholinethan verwendet.

5. Verfahren zur Herstellung von Ameisensäure durch thermische Spaltung von quartären Ammoniumformiaten I nach Anspruch 1, dadurch gekennzeichnet, daß man die Spaltung mit sekundären Formamiden II durchführt, die 50 bis 120 °C niedriger sieden als das im Formiat I enthaltene tertiäre Amin III.

## Claims

1. A process for preparing formic acid by thermal cleavage of quaternary ammonium formates of the general formula I

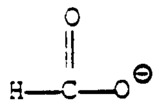 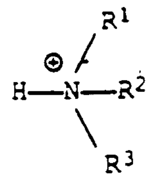 (I)

where

$R^1$, $R^2$ and $R^3$ are $C_1$- to $C_{14}$-alkyl, $C_3$- to $C_8$-cycloalkyl, aryl or $C_7$- to $C_{16}$-aralkyl, or together are 1,4- or 1,5-alkylene which is unsubstituted or substituted one to four times by $C_1$- to $C_4$-alkyl, with the proviso that the sum of the carbon atoms of $R^1$, $R^2$ and $R^3$ in the quaternary ammonium formates I is 7 to 40,

which comprises carrying out the cleavage in the presence of secondary formamides of the general formula II

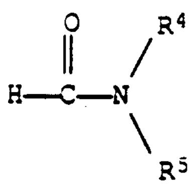 (II)

where

$R^4$ and $R^5$ are $C_2$- to $C_{10}$-alkyl, $C_3$- to $C_8$-cycloalkyl, aryl or $C_7$- to $C_{16}$-aralkyl, or together are 1,4- or 1,5-alkylene which is unsubstituted or substituted one to four times by $C_1$- to $C_4$-alkyl, which formamides boil 30 to 150 °C lower than the tertiary amine of the general formula III

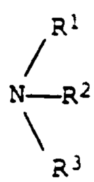 (III)

where $R^1$, $R^2$ and $R^3$ have the abovementioned meanings, contained in the formate I.

2. The process for preparing formic acid by thermal cleavage of quaternary ammonium formates I as claimed in claim 1, wherein the sum of the carbon atoms of $R^1$, $R^2$ and $R^3$ in the quaternary ammonium formates I is 10 to 24.

3. The process for preparing formic acid by thermal cleavage of quaternary ammonium formates I as claimed in claim 1, wherein N-dibutylformamide is used as the secondary formamide II.

4. The process for preparing formic acid by thermal cleavage of quaternary ammonium formates I as claimed in claim 1, wherein quaternary ammonium formates I of the tertiary amines III trihexylamine, triheptylamine, trioctylamine, dimethyltetradecylamine, N-hexylmorpholine or N,N'-dimorpholinoethane

are used.

5. The process for preparing formic acid by thermal cleavage of quaternary ammonium formates I as claimed in claim 1, wherein the cleavage is carried out with secondary formamides II which boil 50 to 120 °C lower than the tertiary amine III contained in the formate I.

**Revendications**

1. Procédé de préparation d'acide formique par dissociation thermique de formiates d'ammonium quaternaires de formule générale I

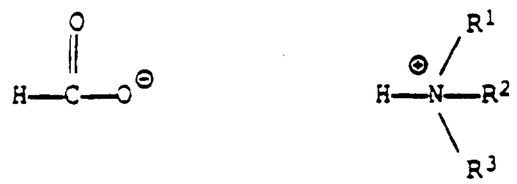

(I),

dans laquelle

$R^1$,$R^2$,$R^3$ représentent chacun un reste alkyle en $C_1$-$C_{14}$,cycloalkyle en $C_3$-$C_8$, aryle ou aralkyle en $C_7$-$C_{16}$, ou forment ensemble un groupement 1,4- ou 1,5-alkylène éventuellement substitué une à quatre fois par un reste alkyle en $C_1$-$C_4$, étant spécifié que la somme des nombres d'atomes de carbone de $R^1$, $R^2$ et $R^3$ dans les formiates d'ammonium quarternaires I est comprise entre 7 et 40,

caractérisé en ce que l'on effectue la dissociation en présence de formamides secondaires de formule générale II

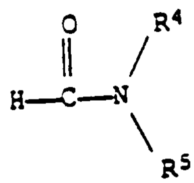

(II),

dans laquelle

$R^4$ et $R^5$ représentent chacun un reste alkyle en $C_2$-$C_{10}$, cycloalkyle en $C_3$-$C_8$, aryle ou aralkyle en $C_7$-$C_{16}$, ou forment ensemble un groupement 1,4- ou 1,5-alkylène éventuellement substitué une à quatre fois par un reste alkyle en $C_1$-$C_4$,

dont le point d'ébullition est inférieur de 30 à 150 °C à celui de l'amine tertiaire de formule générale III contenue dans le formiate I

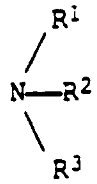

(III),

dans laquelle $R^1$, $R^2$ et $R^3$ ont les significations données ci-dessus.

2. Procédé de préparation d'acide formique par dissociation thermique de formiates d'ammonium quaternaires I selon la revendication 1, caractérisé en ce que la somme des nombres d'atomes de carbone de $R^1$, $R^2$ et $R^3$ dans les formiates d'ammonium quaternaires I est comprise entre 10 et 24.

3. Procédé de préparation d'acide formique par dissociation thermique de formiates d'ammonium quaternaires I selon la revendication 1, caractérisé en ce que l'on utilise du N-dibutylformamide comme formamide secondaire II.

4. Procédé de préparation d'acide formique par dissociation thermique de formiates d'ammonium quaternaires I selon la revendication 1, caractérisé en ce que l'on utilise des formiates d'ammonium quaternaires I des amines tertiaires III trihexylamine, triheptylamine, trioctylamine, diméthyltétradécylamine, N-hexylmorpholine ou N,N'-dimorpholine-éthane.

5. Procédé de préparation d'acide formique par dissociation thermique de formiates d'ammonium quaternaires I selon la revendication 1, caractérisé en ce que l'on effectue la dissociation avec des formamides secondaires II dont le point d'ébullition est inférieur de 50 à 120 °C à celui de l'amine tertiaire III contenue dans le formiate I.